Europäisches Patentamt

European Patent Office

Office européen des brevets

Veröffentlichungsnummer: **0 285 835**

**A2**

## EUROPÄISCHE PATENTANMELDUNG

Anmeldenummer: 88103679.2

Int. Cl.4 **C11C 3/12**

Anmeldetag: 09.03.88

Priorität: 16.03.87 DE 3708430

Veröffentlichungstag der Anmeldung:
**12.10.88 Patentblatt 88/41**

Benannte Vertragsstaaten:
**AT BE CH DE ES FR GB IT LI LU NL SE**

Anmelder: **Henkel Kommanditgesellschaft auf Aktlen**
**Postfach 1100 Henkelstrasse 67**
**D-4000 Düsseldorf-Holthausen(DE)**

Erfinder: **Carduck, Franz-Josef, Dr.**
**Landstrasse 18**
**D-5657 Haan(DE)**
Erfinder: **Falbe, Jürgen, Prof. Dr.**
**Linnéplatz 14**
**D-4040 Neuss 1(DE)**
Erfinder: **Fleckenstein, Theo, Dr.**
**Pfitznerstrasse 9**
**D-4010 Hilden(DE)**
Erfinder: **Göbel, Gerd, Dr.**
**Lindenstrasse 10**
**D-4006 Erkrath(DE)**
Erfinder: **Pohl, Joachim, Dr.**
**Leinenweberweg 23**
**D-4000 Düsseldorf(DE)**

### Verfahren zur Direkthydrierung von Butterfett.

Bei der katalytischen Direkthydrierung von Butterfett wird dieses zusammen mit Wasserstoff bei Drucken von 20 bis 300 bar und Temperaturen von 180 bis 250°C bei Molverhältnissen von Wasserstoff : Fettsäurerest im Butterfett von 10 : 1 bis 500 : 1 kontinuierlich über Katalysatoren umgesetzt, die 30 bis 40 Gew.-% Kupfer, 23 bis 30 Gew.-% Chrom 1 bis 10 Gew.-% Mangan, 1 bis 10 Gew.-% Silicium und 1 bis 7 Gew.-% Barium - Gew.-% bezogen jeweils auf oxidische Katalysatormasse - sowie gewünschtenfalls weitere Übergangsmetalle in Form ihrer Oxide enthalten und nach dem Calcinieren der die Katalysatormasse bildenden Komponenten mit 1 bis 10 Gew.-% - bezogen auf oxidischen Katalysator - wenigstens eines Binders neben 1 bis 10 Gew.-% an Graphit zu stückigen und/ oder gekörnten Formlingen umgewandelt und mit Wasserstoff oder einem Wasserstoff enthaltenden Gasgemisch aktiviert worden sind.

Dabei entstehen ohne vorherige Abtrennung der kürzerkettigen Fettsäuren die Fettalkohole, Oxo-und Hydroxyfettalkohole entsprechend der natürlichen Fettsäurezusammensetzung und das gewünschte Wertprodukt Propylenglycol.

EP 0 285 835 A2

## Verfahren zur Direkthydrierung von Butterfett

Die Erfindung betrifft ein neues Verfahren zur katalytischen Direkthydrierung von Butterfett ohne vorherige Abtrennung kurzkettiger Fettsäuren bei erhöhten Reaktionstemperaturen unter Verwendung stückiger und/oder gekörnter Kupferchromit enthaltender Katalysatoren im Druckbereich von 20 bis 300 bar zu Fettalkoholen, Oxo-und Hydroxyfettalkoholen entsprechend der natürlichen Fettsäurezusammensetzung und Propylenglycol als Wertprodukt.

Durch Ausschmelzen von Butter, Abtrennen der wäßrigen Phase und Erhitzen auf 100 bis 105 °C erhält man Butterschmalz, unter dem man 99,3 %iges Butterfett versteht, das höchstens 0,5 % Wasser und praktisch keine Lactose, Casein und Salze enthält. (Römpp's Chemie Lexikon, 8. Auflage, S. 544 und dort genannte Literatur).

Fettalkohole, d.h. überwiegend lineare, monofunktionelle Alkohole mit Kettenlängen von 8 und mehr C-Atomen, sowie ihre Herstellung sind in der Literatur ausführlich beschrieben, beispielsweise in Ullmanns Encyklopädie der technischen Chemie, 4. Auflage, Band 11, Seiten 427 bis 445. Ein bevorzugtes Ausgangsmaterial zu ihrer Gewinnung sind die in Fetten und/oder Ölen natürlichen Ursprungs vorkommenden Fettsäuren bzw. Fettsäuregemische, die durch katalytische Hydrierung in Fettalkohole entsprechender Kettenlänge umgewandelt werden können. Durch den Einsatz der zu reduzierenden Fettsäuren in Form ihrer Methylester werden insbesondere die Katalysatoren vor einem aggressiven Angriff durch die freie Carboxylgruppe geschützt, so daß in großtechnischen Verfahren für hinreichend lange Zeiträume mit befriedigenden Raumzeitausbeuten gearbeitet werden kann. Demgemäß wird die überwiegende Menge nativer Fettalkohole heute aus Fettsäuremethylestern nach dem Gasphasenhydrierverfahren hergestellt, bei dem die destillierten Methylester über fest angeordnete, kupferhaltige Mischoxid-Katalysatoren, wie beispielsweise Kupferchromit-Spinellkatalysatoren, in dampfförmigem Zustand zusammen mit einem großen Überschuß an Wasserstoff bei Temperaturen von oberhalb 200°C und Drucken von etwa 250 bis 300 bar geleitet werden.

Die auf feuchtem Wege durch gemeinsame Fällung hergestellten Kupfer-Mischoxid-Katalysatoren werden als stückige Kontakte oder Strangpreßlinge benutzt und vor Gebrauch meist in der Anlage reduziert.

In der einschlägigen Patentliteratur werden daher häufig Fettsäureester, insbesondere Fettsäuremethylester und freie Fettsäuren gleichzeitig als Einsatzmaterialien für die Hydrierungsreaktion zu gesättigten und/oder ungesättigten Fettalkoholen verwendet. Verwiesen sei beispielsweise auf die DE-PSen 965 236, 10 05 497, 25 13 377 und 26 13 226. Für die großtechnische praktische Verwertung sind die genannten Vorschläge durchaus unterschiedlich zu bewerten, je nachdem, ob die Fettsäureester oder die freien Fettsäuren als Ausgangsmaterial der Hydrierung verwendet werden.

Butter stellt mit seiner Fettsäurezusammensetzung einen Talgersatzstoff dar. Bei konventioneller Verarbeitung von Butter bzw. Butterfett, sowohl bei der Umesterung als auch bei der Fettspaltung treten wegen des hohen Anteils kurzkettiger Fettsäuren mit 4 bis 10 C-Atomen verfahrenstechnische Schwierigkeiten auf, die zu unwirtschaftlichen Hydrierverfahren führen. Üblicherweise werden die kurzkettigen Fettsäuren als Methylester abgetrennt. Hierbei treten ebenso wie bei der Verarbeitung über die Fettspaltung unangenehme Geruchsemissionen durch die kurzkettigen Fettsäuren auf. Zudem bereitet die hohe Löslichkeit der Fettsäuren im Spaltwaser praktische Probleme.

Weitere Probleme treten bei der Abtrennung des Methanols von den kurzkettigen Fettsäuren auf, da sich die Siedepunkte teilweise überschneiden und somit ein Teil des Methanols bei der Aufarbeitung verlorengeht.

Aufgabe der vorliegenden Erfindung war es, ein Verfahren zur katalytischen Direkthydrierung von Butterfett ohne vorherige Abtrennung der kürzerkettigen Fettsäuren unter Verwendung stückiger und/oder gekörnter, Kupferchromit enthaltender Katalysatoren zur Verfügung zu stellen, mit dem in hoher Ausbeute das Wertprodukt Propylenglycol und die Fettalkohole, Oxo-und Hydroxyfettalkohole entsprechend der natürlichen Fettsäurezusammensetzung erhalten werden.

Der für die Umsetzung verwendete heterogene Übergangsmetall-Katalysator sollte mit hoher Aktivität und Se lektivität zu den gewünschten Produkten, insbesondere Propylenglycol führen, ohne daß Folgereaktionen in nennenswertem Maß zu einer Verminderung der Produktausbeute beitragen. Damit sollte die Wirtschaftlichkeit des Verfahrens im Vergleich zum Stand der Technik verbessert werden.

Es wurde nun überraschend gefunden, daß es mit bestimmten, Kupferchromit als Hauptbestandteil enthaltenden Katalysatoren mit hoher Aktivität und Selektivität unter langer Standzeit der Katalysatoren möglich ist, die Direkthydrierung von Butterfett zu Fettalkoholen und Propylenglycol so zu steuern, daß die Bildung von Kohlenwasserstoffen weitgehend unterdrückt wird und gleichzeitig in hoher Ausbeute das Wertprodukt Propylenglycol entsteht.

Die Erfindung betrifft ein Verfahren zur katalytischen Direkthydrierung von Butterfett ohne vorherige Abtrennung kurzkettiger Fettsäuren bei erhöhten Reaktionstemperaturen unter Verwendung stückiger und oder gekörnter, Kupferchromit als Hauptbestandteil enthaltender Katalysatoren zu Fettalkoholen, Oxo- und Hydroxyfettalkoholen entsprechend der natürlichen Fettsäurezusammensetzung und Propylenglycol, das dadurch gekennzeichnet ist, daß man Butterfett zusammen mit Wasserstoff bei Drucken von 20 bis 300 bar und Temperaturen von 180 bis 250°C bei Molverhältnissen von Wasserstoff : Fettsäurerest im Butterfett von 10 : 1 bis 500 : 1 kontinuierlich über Katalysatoren umsetzt, die 30 bis 40 Gew.-% Kupfer, 23 bis 30 Gew.-% Chrom, 1 bis 10 Gew.-% Mangan, 1 bis 10 Gew.-% Silicium und 1 bis 7 Gew.-% Barium - Gew.-% ‚bezogen jeweils auf oxidische Katalysatormasse - sowie gewünschtenfalls weitere Übergangsmetalle in Form ihrer Oxide ent halten und nach dem Calcinieren der die Katalysatormasse bildenden Komponenten mit 1 bis 10 Gew.-% - bezogen auf oxidischen Katalysator - wenigstens eines Binders neben 1 bis 10 Gew.-% Graphit zu stückigen und/oder grobkörnigen Formlingen umgewandelt und mit Wasserstoff oder einem Wasserstoff enthaltenden Gasgemisch aktiviert worden sind.

Gemäß einer bevorzugten Ausführungsform wird die Hydrierung in einem Druckbereich von 100 bis 300 bar durchgeführt.

Als Ausgangsstoff für das erfindungsgemäße Verfahren zur Hydrierung kommt Butter in Frage, die einfach oder mehrfach ungesättigte Fettsäuren enthält, die mit Glycerin verestert sind, wobei die Fettsäurereste in den Glyceridmolekülen gleiche oder unterschiedliche Sättigungsgrade und Kettenlängen aufweisen können.

Butter bzw. Butterfett wird nach Vorreinigung, Entwässerung und Entschwefelung als Triglycerid mit Wasserstoff in einem Rieselbettreaktor mit fest angeordnetem Katalysator umgesetzt. Als Produkt wird ein Gemisch von Fettalkoholen, Oxo-und Hydroxyfettalkoholen entsprechend der natürlichen Fettsäurezusammensetzung erhalten. Ungesättigte C-C-Doppelbindugen werden abgesättigt. Unter Abspaltung von Wasser wird aus dem gebundenen Glycerin Propylenglycol gebildet. Der Umsetzungsgrad beträgt 99 %.

Der Hydrierablauf fällt als farblose klare Flüssigkeit ohne den typischen ranzigen Geruch an und wird nach an sich bekannten Verfahren zunächst getrocknet und von leicht siedenden Bestandteilen befreit.

Das Produktgemisch wird anschließend destillativ in einem Vorlauf (C 4 - C 10 ), Hauptlauf (C 12 - C 18) und einem Rückstand aufgetrennt, wobei der Rückstand teilweise recyclisiert wird.

Der Hauptlauf entspricht in seiner C-Kettenverteilung etwa der Zusammensetzung von Talgfettalkohol, d.h. Fettalkoholen mit im wesentlichen 14, 16 und 18 C-Atomen.

Der Vorlauf wird zur Abtrennung des Propylenglycols einer Extraktion mit Wasser unterzogen und destillativ aufgearbeitet.

Der Vorteil der vorliegenden Erfindung besteht darin, daß bei Verwendung des erfindungsgemäßen Verfahrens der Direkthydrierung von Butterfett die Stufe der Umesterung bzw. Fettspaltung von Butterfett umgangen wird. Dadurch entfällt die Notwendigkeit, die in der Butter enthaltenen Anteile von ca. 10 % 4 - 10 C-Atome enthaltende Fettsäuren als Methylester oder als freie Fettsäuren abzutrennen. Darüberhinaus wird ein erhöhter Reinigungsaufwand, wie er bei der Abtrennung von Methanol bei der Methylesterhydrierung erforderlich ist, vermieden. Ebenfalls treten die bei der Verarbeitung über die Fettspaltung üblichen unangenehmen Geruchsemissionen und Probleme der hohen Löslichkeit der kurzkettigen Fettsäuren im Spaltwasser nicht auf. Zudem erhält man in hoher Ausbeute das Wertprodukt Propylenglycol.

Nach dem erfindungsgemäßen Verfahren wird die katalytische Direkthydrierung in Gegenwart eines Katalysators durchgeführt, der - bezogen auf die oxidische Katalysatormasse - 30 bis 40 Gew.-% Kupfer, 23 bis 30 Gew.-% Chrom, 1 bis 10 Gew.-% Mangan, 1 bis 7 Gew.-% Barium und gegebenenfalls weitere Übergangsmetalle enthält. Die genannten Metalle liegen nach der Herstellung der Katalysatormassen, die auf an sich aus dem Stand der Technik bekannte Art und Weise erfolgt, in Form ihrer Oxide vor. Die Oxidbildung erfolgt, wie aus dem Stand der Technik bekannt, im Verlauf des sogenannten "Calcinierens", d.h. durch thermische Zersetzung von Mineralsalzen der jeweiligen Metalle.

Im erfindungsgemäßen Verfahren setzt man zur Direkthydrierung von Butterfett einen Katalysator ein, der, bezogen auf die oxidische Katalysatormasse, 1 bis 10 Gew.-% $SiO_2$ enthält.

In einer bevorzugten Ausführungsform des erfindungsgemäßen Verfahrens hydriert man kontinuierlich Butterfett unter Verwendung eines Katalysators, der vorteilhafterweise 32 bis 38 Gew.-% Kupfer, bezogen auf die oxidische Katalysatormasse, enthält. Ebenso kann es bevorzugt sein, die Menge an Chrom in dem verwendeten Katalysator auf einen Bereich von 26 bis 29 Gew.-%, die Menge an Mangan auf einen Bereich von 1 bis 10 Gew.-%, die Menge an Barium auf einen Bereich von 1,5 bis 3 Gew.-% oder die Menge an Silicium auf einen Bereich von 1,5 bis 3 Gew.-%, jeweils bezogen auf die oxidische Katalysatormasse vor der Aktivierung, einzustellen. In einer besonders bevorzugten Ausführungsform wird zur katalytischen Direkthydrierung von Butterfett ein Katalysator verwendet, der 36 Gew.-% Kupfer, 29 Gew.-% Chrom, 2,5

3

Gew.-% Mangan, 1,7 Gew.-% Barium und 1,0 Gew.-% Silicium, jeweils bezogen auf die oxidische Katalysatormasse vor der Aktivierung, und gegebenenfalls weitere Übergangsmetalle in Form ihrer Oxide enthält. Mit derartigen Katalysatoren lassen sich erhebliche Aktivitätssteigerungen erzielen. Aus diesem Grunde ist die Verwendung eines derartigen Katalysators in dem erfindungsgemäßen Verfahren als besonders bevorzugt anzusehen.

In einer weiteren bevorzugten Ausführungsform des Verfahrens kann zur katalytischen Direkthydrierung von Butterfett ein Katalysator verwendet werden, der zusätzlich zu den oben genannten Mengen an Kupfer, Chrom, Mangan, Barium und Silicium weitere Übergangsmetalle in Form ihrer Oxide enthält. So kann ein Katalysator eingesetzt werden, der zusätzlich zu den genannten Metallen je 1 bis 5 Gew.-%, bevorzugterweise 2 bis 3 Gew.-%, Zirkon und/oder Cer enthält. Dabei ist es möglich, den erfindungsgemäß verwendeten Katalysatoren eines der genannten Übergangsmetalle in Form seiner Oxide oder auch mehrere der genannten Übergangsmetalle in Form ihrer Oxide in beliebigen Mischungen miteinander zuzusetzen. Die Verwendung derartiger, zusätzlich dotierter Katalysatoren in dem Verfahren führt insbesondere bei der Hydrierung im Rieselbett zu einer erheblichen Erhöhung der Aktivität und Selektivität der Katalysatoren in bezug auf die Ausbeute an Propylenglycol.

Erfindungsgemäße Katalysatoren können 1 bis 10 Gew.-% an Graphit zur verbesserten Verarbeitbarkeit der Granulat-und/oder Extrudatkörper enthalten. Vorzugsweise wird eine Menge von 5 Gew.-% Graphit vor der Granulierung dem calcinierten pulverförmigen Material zugesetzt und mit diesem innig vermischt.

Erfindungsgemäß wurde eine Verbesserung des Verfahrens dadurch erreicht, daß der verwendete, die oben genannten Metalle in Form ihrer Oxide und Graphit enthaltende Katalysator unter Einsatz von 1 bis 10 Gew.-% eines oder mehrerer Binder in Granulat-oder Extrudatform gebracht wird. Bevorzugt wird dabei ein Einsatz von 10 Gew.-% eines oder mehrerer Binder. Als solche kommen für diesen Zweck aus dem Stand der Technik bekannte Verbindungen in Frage, wobei in dem erfindungsgemäß verwendeten Katalysator entweder ein oder auch mehrere Binder verwendet werden können. Besonders hat sich der Einsatz eines oder mehrerer Binder aus Polyvinylacetat und Methylmethacrylat bewährt. Im Gegensatz zu zahlreichen, aus dem Stand der Technik bekannten, schlecht rieselfähigen Katalysatormaterialien konnte für das erfindungsgemäße Verfahren ein Katalysator in Granulat-oder Extrudatform zur Verfügung gestellt werden, dessen aufgelockerte, poröse Struktur zur Erhöhung der Aktivität und Selektivität des Katalysators bei der Direkthydrierung von Butterfett, insbesondere im Rieselbett in erheblichem Maße beiträgt. Bevorzugt wird als Binder für die Herstellung der Katalysatorgranulate oder Katalysatorextrudate Polyvinylacetat, wobei zur Katalysatorherstellung beispielsweise 40 Gew.-%ige Polyvinylacetat-Suspensionen verwendet werden, die im Handel erhältlich sind. Die calcinierten pulverförmigen Katalysatormaterialien werden nach guter Durchmischung mit derartigen Polyvinylacetat-Suspensionen in kleinen Mengen versetzt und bis zum Beginn des Aufbaus von Agglomeratkörnern gemischt. Im Anschluß daran wird das Agglomerate enthaltende Pulver beispielsweise in einem Lochwalzengranulator zu kleinen Granulaten verdichtet, wobei dieser Prozeß an sich aus dem Stand der Technik bekannt ist. Auf eben falls an sich bekannte Weise werden die Granluate getrocknet, wobei sich Restfeuchten von 10 bis 15 % einstellen lassen. Die aus diesem Vorgang resultierenden Granulate werden gesiebt, wobei für das erfindungsgemäße Verfahren Kornfraktionen bestimmter Korngröße ausgesiebt werden. Bei der Anwendung des erfindungsgemäßen Verfahrens zur katalytischen Hydrierung von Fettsäuremethylestergemischen in der Rieselfahrweise werden mit Vorteil Katalysator-Kornfraktionen eingesetzt, deren Korngröße im Bereich von 0,6 bis 3 mm liegt.

Die Katalysatoren können auch in Tablettenform, beispielsweise der Größe 4 $\times$ 4 mm, gepreßt werden. Zur Härtung dieser Tabletten werden diese 6 h bei einer Temperatur von 200°C an der Luft getempert. Die nach der BET-Methode (Z. Anal. Chem. 238 (1968), 187-193) bestimmte spezifische Oberfläche betrug 40 ± 10 m²/g.

Die in dem erfindungsgemäßen Verfahren zur Direkthydrierung von Butterfett verwendbaren granulierten Katalysatoren weisen eine spezifische Oberfläche im Bereich von 30 bis 50 m²/g auf. Die beschriebene Art der Vorgranulierung führt zu einer speziellen, aufgelockerten Porenstruktur, die sich in einer Erhöhung des Porennutzungsgrades auswirkt.

Im Verlauf der Untersuchungen zu dem erfindungsgemäßen Verfahren zur Direkthydrierung von Butterfett hat sich gezeigt, daß es besonders bevorzugt ist, Butterfett in Gegenwart eines Katalysators mit Wasserstoff umzusetzen, dessen Granulat-oder Extrudatkörper einen Durchmesser von 1 bis 6 mm und eine Länge von 1 bis 6 mm aufweisen. Derartige Granulat-bzw. Extrudat körper (Tabletten) zeigen eine ausgezeichnete Aktivität und Selektivität bei der Umsetzung von Butterfett mit Wasserstoff und lassen sich zudem ohne Probleme von den Reaktionsprodukten abtrennen. Außerdem sind die mit diesen Katalysatoren erzielbaren Standzeiten deutlich besser als die Standzeiten der aus dem Stand der Technik bekannten Katalysatoren, die zudem den Nachteil aufwiesen, daß sie zum Teil bei der Reaktion zerfielen und dadurch nur unter großen Problemen von den Reaktionsprodukten abtrennbar waren.

4

Ein weiterer Faktor, der die Aktivität bzw. Selektivität der erfindungsgemäß eingesetzten Katalysatoren stark beeinflußt, ist das Porenvolumen der Katalysator-Formkörper. Es zeigte sich, daß das Porenvolumen der erfindungsgemäß verwendbaren Katalysatoren in einem Optimalbereich liegen muß, um optimale Ergebnisse zu erbringen. In einer bevorzugten Ausführungsform des erfindungsgemäßen Verfahrens werden metallhaltige Katalysatoren verwendet, deren Porenvolumen im Bereich von 0,4 bis 0,6 $cm^3$ g liegt. Vorteilhafterweise trägt ein Porenvolumen in diesem Bereich ebenfalls zu einer Erhöhung der Aktivität und Selektivität der Hydrierkatalysatoren bei: Sowohl im Rieselbettreaktor als auch im Sumpfphasenreaktor lassen sich hohe Aktivitäten und Selektivitäten erzielen; gleichzeitig wiesen derartige Katalysatoren in dem erfindungsgemäßen Verfahren eine ausgesprochen hohe Standzeit auf und führten nicht zu Problemen bei der Trennung von Katalysator und Reaktionsprodukten.

Die in dem erfindungsgemäßen Verfahren verwendeten Katalysatoren werden üblicherweise vor ihrem Einsatz mit Wasserstoff oder einem Wasserstoff enthaltenden Gasgemisch aktiviert. Aus ökonomischen Gründen wird mit Vorteil zur Aktivierung der Katalysatormassen ein Gasgemisch verwendet, das überwiegend aus einer Stickstoff-Wasserstoff-Gasmischung besteht. Vorteilhafterweise kann eine derartige Aktivierung - wie aus dem Stand der Technik bekannt - so durchgeführt werden, daß die Katalysatormassen nach der Herstellung im Stickstoffstrom bei erhöhter Temperatur getrocknet werden und dem trocknenden Gas in steigenden Mengen Wasserstoff zur Aktivierung beigemischt wird. Dabei kann der Wasserstoffanteil in dem aktivierenden Gasgemisch im Bereich von 0,1 bis 10 Vol.-% liegen. Die Aktivierung der Katalysatoren kann dabei sowohl in situ als auch in von dem Reaktionsgefäß getrennten Gefäßen durchgeführt werden.

Die Reaktionstemperaturen der Direkthydrierung von Butterfett gemäß der vorliegenden Erfindung liegen im Bereich von 180 bis 250°C, bevorzugt im Bereich von 200 bis 240°C. Bei der Temperaturführung der Reaktion ist allgemein zu beachten, daß die Hydrierung zu entsprechenden Fettalkoholen, Oxo-und Hydroxyfettalkohole entsprechend der natürlichen Fettsäurezusammensetzung, eine exotherme chemische Reaktion ist. Bei der Temperaturführung muß also darauf geachtet werden, daß nach "Anspringen" der Reduktion die entstehende Reaktionswärme in üblicher Weise abzuführen ist.

Das erfindungsgemäße Verfahren zur Direkthydrierung von Butterfett ist außerdem dadurch gekennzeichnet, daß man ein Molverhälntis von Wasserstoff zu Fettsäurerest im Butterfett-Substrat von 10 : 1 bis 500 : 1 einstellt. Dies bedeutet, daß die Durchsatzmenge an Wasserstoffgas, gemessen in Mol/Stunde, 10- bis 500mal höher liegt als die Durchsatzmenge an Butterfett, gemessen in Mol Fettsäurerest/Stunde.

Die Vorteile des erfindungsgemäßen Verfahrens sind darin zu sehen, daß durch eine Optimierung sowohl der chemischen Zusammensetzung als auch des physikalischen Aufbaus der verwendeten Katalysatoren auf Kupferchromit-Basis das Verfahren zur Herstellung von nativen Fettalkoholen vereinfacht wird, da hier - im Gegensatz zur Hydrierung von Fettsäuremethylestern - keine aufwendige Abtrennung des Methanol von kurzkettigen Fettsäuremethylestern notwendig ist.

Der Einsatz des erfindungsgemäßen Katalysators in Verbindung mit der beschriebenen Verfahrensweise erlaubt die Herstellung von Fettalkoholen, Oxo-und Hydroxyfettalkohole entsprechend der natürlichen Fettsäurezusammensetzung von Butterfett, ohne vorherige Abtrennung der kurzkettigen Fettsäuren. Darüberhinaus wird Propylenglycol als wertvolles Nebenprodukt erhalten.

In den nachfolgenden Beispielen, in denen die Erfindung näher erläutert wird, sind alle Prozentangaben in Gewichtsprozent, wenn nicht ausdrücklich anders angegeben.

## Ausführungsbeispiel

Herstellung eines Katalysators:

Bei Temperaturen von 30 bis 90°C wurden 84,93 g $Ba(NO_3)_2$, 3493 g $Cu(NO_3)_2$ . 2 $H_2O$, 294,43 g Mn-$(NO_3)_2$ . 4 $H_2O$ und 62,3 g $SiO_2$ in Form eines 40 Gew.%igen Kieselsäuresols in 9 l entsalztem Wasser unter starkem Rühren gelöst. In einem zweiten Behälter wurden 1639 g $CrO_3$ in 9 l entsalztem Wasser unter den gleichen Bedingungen gelöst und anschließend 3650 g einer 25 %igen Ammoniaklösung hinzugegeben. Danach wurde die Barium, Mangan und Kupfer enthaltende Lösung bei Temperaturen von 30 bis 90°C in die vorgelegte Ammoniumchromatlösung gepumpt, wobei aus der Lösung ein Gemenge von Bariumchromat, Manganhydroxid, Siliciumhydroxid und Kupferchromat ausfiel. Die Fällung war beendet bei Absinken des pH-Wertes unter 7.

Das Präzipitat wurde in einer Rahmenfilterpresse abfiltriert und mit entsalztem Wasser nitratfrei gewaschen. Der Filterkuchen wurde über Nacht bei 90 bis 120°C getrocknet und danach in einer Schneidmühle zu grobem Pulver gemahlen. Das resultierende Chromatpulver wurde im Drehrohrofen bei Temperaturen von 300 bis 500°C thermisch zum Chromit zersetzt ("calciniert"). Das calcinierte, pulverförmige Material hatte folgende chemische Zusammensetzung:

Cu: 38 ± 0,5 %;

Cr: 29 ± 0,5 %;

Mn: 2,5 ± 0,5 %;

Ba: 1,9 ± 0,5 % und

Si: 1 ± 0,3 %.

Einem Liter des Pulvers wurden 5 Gew.-% Graphit zugesetzt und im Lödige-Mischer 15 min durchmischt. Anschließend wurden 10 Gew.-% einer 40 gew.-%igen Polyvinylacetatsuspension zugegeben und kurz bis zum Aufbaubeginn von Agglomeraten durchgemischt. Im Anschluß daran wurde das Pulver in einem Lochwalzengranulator zu kleinen Granulaten verdichtet, auf eine Restfeuchte von 10 bis 15 % getrocknet und zu einer 0,6-bis 3 mm-Kornfraktion gesiebt.

Das Pulver hatte ausgezeichnete Fließeigenschaften und konnte auf einer Rundläufer-Tablettenmaschine zu Tabletten von 3 bis 6 mm Durchmesser und 2 bis 4 mm Höhe verpreßt werden.

Nach der Härtung (6h, 200°C, an der Luft) resultierten Tabletten mit einer spezifischen Oberfläche (bestimmt nach BET) von 40 ± 10 $m^2/g$ und einem Porenvolumen von 0,4 bis 0,6 $cm^3/g$.

Beispiel 1

Als Ausgangsmaterial wurde salzfreie deutsche Markenbutter verwendet. Die Butter wurde aufgeschmolzen und die Triglyceride von Wasser und einer Emulsionsphase getrennt.

0,5 l des gemäß dem Ausführungsbeispiel hergestellten und granulierten Katalysators mit einer mittleren Korngröße von 1 mm wurden in ein Reaktionsrohr mit dem Volumen von einem Liter und einem Innendurchmesser von 25 mm eingefüllt und zur gleichmäßigen Verteilung der flüssigen Phase mit 2 mm Glaskugeln überschichtet.

Nach Trocknung und Reduktion des Katalysators in einem Stickstoff/Wasserstoffstrom (Wasserstoffkonz. (max.) 1 %; Temperatur (max.) 200 °C) wurde bei 250 bar und 200 bis 220 °C kontinuierlich Wasserstoff im Gleichstrom mit Butterfett von oben durch den Reaktor geleitet und das flüssige, klare Reaktionsprodukt, das sich bei Raumtemperatur verfestigte, in einem Abscheidesystem aufgefangen. Die Reaktionsbedingungen und die Produktanalysen sind wie folgt zusammengefaßt:

Versuchsergebnisse

```
Druck: 250 bar           Säurezahl  =  0,6
Schwefelgehalt: 16 ppm
```

| | |
|---|---|
| Reaktionstemperatur (°C) | 220 |
| LHSV* (l × l$^{-1}$ × h$^{-1}$) | 1,0 |
| Wasserstoff-Durchsatz (Nm$^3$/h) | 10 |
| Produkt-Verseifungszahl | 1,0 |

*) LHSV = liquid hourly space velocity

Die Analyse der C-Kettenverteilung der Fettsäuren ergab:

Die GC-Analyse ergab für den Fettsäureanteil folgende Kettenlängenverteilung

C 4 gesättigt     3,2 %
C 6 gesättigt     2,7 %
C 8 gesättigt     1,4 %
C 10 gesättigt     3,0 %
C 12 gesättigt     3,8 %
C 14 gesättigt     11,0 %
C 15 gesättigt     1,1 %
C 16 gesättigt     27,6 %
C 16 einfach ungesättigt     1,5 %
C 17 gesättigt     0,5 %
C 18 gesättigt     10,0 %
C 18 einfach ungesättigt     23,1 %
C 18 zweifach ungesättigt     1,4 %
C 18 dreifach ungesättigt     1,2 %
Rest * 8,5 %

Es wurde ein farbloser, klarer Hydrierablauf, der sich bei Zimmertemperatur verfestigte, erhalten. Die Verseifungszahlen lagen unter 1,0.

Neben den durchgehärteten Fettalkoholen wurden 8,8 % Propylenglycol (entsprechend 86 % der Theorie) und 0,19 % Kohlenwasserstoffe gefunden.

Beispiel 2:

Als Einsatzmaterial wurde Butterfett (99,8 %ig) verwendet, das durch eine Vorbehandlung mit Bleicherde-'Aktivkohle mit 2 % einer Mischung aus Floridin und 10 % Aktivkohle bei 90° C im Verlauf von 0,5 Stunden erhalten wurde.

Die Versuche wurden im Rieselbett mit dem Volumen 0,5 l analog Beispiel 1 durchgeführt, wobei Butterfett nach dem Ausschmelzen und Erhitzen auf Reaktionstemperatur zusammen mit vorerhitztem Wasserstoff im Gleichstrom von oben nach unten durch den Reaktor geführt wurde.

Kennzahlen des Ausgangsproduktes Säurezahl:     1,2
Verseifungszahl:     234
Jodzahl:     29,3
Schwefelgehalt:     4 ppm

Der Fettsäureanteil hatte nach der GC-Analyse folgende C-Kettenverteilung

C 4 gesättigt     1,4 %
C 6 gesättigt     2,8 %
C 8 gesättigt     2,1 %
C 10 gesättigt     4,7 %
C 12 gesättigt     4,7 %
C 14 gesättigt     12,75 %
C 15 gesättigt     1,1 %
C 16 gesättigt     22,8 %
C 16 einfach ungesättigt     7,1 %

*) Der Rest bestand überwiegend aus nicht näher identifizierten verzweigten Fettsäuren.

*) Der Rest bestand überwiegend aus nicht näher identifizierten verzweigten Fettsäuren.

C 18 gesättigt      7.2 %
C 18 einfach ungesättigt      17,8 %
C 18 zweifach ungesättigt      3,6 %
Rest *      11,95 %

Reaktionsbedingungen:
Temperatur 200°C
Druck 250 bar
LHSV 1 l $\times$ l$^{-1}$ $\times$ h$^{-1}$
Wasserstoff:Substrat = 200 : 1 (mol/moläquivalente)

Reaktionsprodukt:

Wasserklarer Hydrierablauf

Kennzahlen: Säurezahl:      0,5
Verseifungszahl:      0,4
Jodzahl:      1
Gehalt an Fettalkoholen:      87,0 %
Propylenglycol:      8,6 %
Kohlenwasserstoffe:      0,01 %

Beispiel 3

Bei einer Reaktionstemperatur von 195°C wurde unter ansonsten identischen Bedingungen wie in Beispiel 2 das gleiche Butterfettausgangsprodukt hydriert, wobei wiederum ein wasserklarer Hydrierablauf erhalten wurde.

Kennzahlen: Säurezahl:      0,5
Verseifungszahl:      1,0
Jodzahl:      1

Gehalt an Fettalkoholen:      86,9 %
Propylenglycol:      9,0 %
Kohlenwasserstoffe:      0,01 %

**Ansprüche**

1. Verfahren zur katalytischen Direkthydrierung von Butterfett ohne vorherige Abtrennung kurzkettiger Fettsäuren bei erhöhten Reaktionstemperaturen unter Verwendung stückiger und/oder gekörnter, Kupferchromit als Hauptbestandteil enthaltender Katalysatoren zu Fettalkoholen, Oxo-und Hydroxyfettalkoholen entsprechend der natürlichen Fettsäurezusammensetzung und Propylenglycol, dadurch gekennzeichnet, daß man Butterfett zusammen mit Wasserstoff bei Drucken von 20 bis 300 bar und Temperaturen von 180 bis 250°C bei Molverhältnissen von Wasserstoff : Fettsäureresten im Butterfett-Einsatz von 10 : 1 bis 500 : 1 kontinuierlich über Katalysatoren umsetzt, die 30 bis 40 Gew.-% Kupfer, 23 bis 30 Gew.-% Chrom, 1 bis 10 Gew.-% Mangan, 1 bis 10 Gew.-% Silicium und 1 bis 7 Gew.-% Barium - Gew.-% bezogen jeweils auf oxidische Katalysatormasse - sowie gewünschtenfalls weitere Übergangsmetalle in Form ihrer Oxide enthalten und nach dem Calcinieren der die Katalysatormasse bildenden Komponenten mit 1 bis 10 Gew.-% - bezogen auf oxidischen Katalysator - wenigstens eines Binders neben 1 bis 10 Gew.-% an Graphit zu stückigen und/oder gekörnten Formlingen umgewandelt und mit Wasserstoff oder einem Wasserstoff enthaltenden Gasgemisch aktiviert worden sind.

2. Verfahren nach Anspruch 1. dadurch gekennzeichnet, daß man bei Drucken von 100 bis 300 bar hydriert.

3. Verfahren nach Ansprüchen 1 und 2, dadurch gekennzeichnet, daß man kontinuierlich Butterfett katalytisch mit Wasserstoff umsetzt.

4. Verfahren nach Ansprüchen 1 bis 3, dadurch gekennzeichnet, daß man kontinuierlich Butterfett unter Verwendung eines 32 bis 38 Gew.-%, bezogen auf die oxidische Katalysatormasse, Kupfer enthaltenden Katalysators mit Wasserstoff umsetzt.

5. Verfahren nach Ansprüchen 1 bis 3, dadurch gekennzeichnet, daß man kontinuierlich Butterfett unter Verwendung eines 26 bis 29 Gew.-%, bezogen auf die oxidische Katalysatormasse, Chrom enthaltenden Katalysators mit Wasserstoff umsetzt.

6. Verfahren nach Ansprüchen 1 bis 3, dadurch gekennzeichnet, daß man kontinuierlich Butterfett unter Verwendung eines 1,5 bis 3 Gew.-%, bezogen auf die oxidische Katalysatormasse, Barium enthaltenden Katalysators mit Wasserstoff umsetzt.

7. Verfahren nach Ansprüchen 1 bis 3. dadurch gekennzeichnet, daß man kontinuierlich Butterfett unter Verwendung eines 1,5 bis 3 Gew.-%, bezogen auf die oxidische Katalysatormasse, Silicium enthaltenden Katalysators mit Wasserstoff umsetzt.

8. Verfahren nach Ansprüchen 1 bis 3, dadurch gekennzeichnet, daß man kontinuierlich Butterfett unter Verwendung eines 32 bis 38 Gew.-%, bezogen auf die oxidische Katalysatormasse, Kupfer, 26 bis 29 Gew.-% Chrom, 1 bis 6 Gew.-% Mangan, 1,5 bis 3 Gew.-% Barium und 1,5 bis 3 Gew.-% Silicium und gegebenenfalls weitere Übergangsmetalle in Form ihrer Oxide enthaltenden Katalysators mit Wasserstoff umsetzt.

9. Verfahren nach Ansprüchen 1 bis 8, dadurch gekennzeichnet, daß man kontinuierlich Butterfett unter Verwendung eines 32 bis 38 Gew.-%, bezogen auf die oxidische Katalysatormasse, Kupfer, 26 bis 29 Gew.-% Chrom, 1 bis 6 Gew.-% Mangan, 1,5 bis 3 Gew.-% Barium und 1,5 bis 3 Gew.-% Silicium und zusätzlich je 1 bis 5, bevorzugt 2 bis 3 Gew.-%, bezogen auf die oxidische Katalysatormasse, Zirkon und/ oder Cer, enthaltenden Katalysators mit Wasserstoff umsetzt.

10. Verfahren nach Ansprüchen 1 bis 8, dadurch gekennzeichnet, daß man kontinuierlich Butterfett unter Verwendung eines Metalle in Form ihrer Oxide enthaltenden, unter Einsatz von 10 Gew.-%, bezogen auf die oxidische Katalysatormasse, eines oder mehrerer Binder aus Polyvinylacetat und Methylmethacrylat in Granulat-oder Extrudatform gebrachten Katalysators mit Wasserstoff umsetzt.

11. Verfahren nach Ansprüchen 1 bis 10, dadurch gekennzeichnet, daß man kontinuierlich Butterfett unter Verwendung eines Metalle in Form ihrer Oxide enthaltenden Katalysators mit Wasserstoff umsetzt, der eine Korngröße im Bereich von 0,6 bis 3 mm aufweist.

12. Verfahren nach Ansprüchen 1 bis 10, dadurch gekennzeichnet, daß man kontinuierlich Butterfett unter Verwendung eines Katalysators mit Wasserstoff umsetzt, dessen Granulat-oder Extrudatkörper einen Durchmesser von 1 bis 6 mm und eine Länge von 1 bis 6 mm aufweisen.

13. Verfahren nach Ansprüchen 1 bis 12, dadurch gekennzeichnet, daß man kontinuierlich Butterfett unter Verwendung eines Katalysators mit Wasserstoff umsetzt, der eine spezifische Oberfläche im Bereich von 30 bis 50 $m^2/g$ aufweist.

14. Verfahren nach Ansprüchen 1 bis 13, dadurch gekennzeichnet, daß man kontinuierlich Butterfett unter Verwendung eines Katalysators mit Wasserstoff umsetzt, der ein Porenvolumen im Bereich von 0,4 bis 0,6 $cm^3/g$ aufweist.

15. Verfahren nach Ansprüchen 1 bis 14, dadurch gekennzeichnet, daß man kontinuierlich Butterfett unter Verwendung eines Katalysators mit Wasserstoff umsetzt, der mit einem 0,1 bis 10 Vol.-% Wasserstoff enthaltenden $N_2$-/$H_2$-Gasgemisch aktiviert wurde.

16. Verfahren nach Ansprüchen 1 bis 15, dadurch gekennzeichnet, daß man kontinuierlich Butterfett unter Verwendung eines Metalle in Form ihrer Oxide enthaltenden, granulat-oder extrudatförmigen, mit einem Wasserstoff enthaltenden Gasgemisch aktivierten Katalysators bei Reaktionstemperaturen von 200 bis 240°C mit Wasserstoff umsetzt.